# EUROPEAN PATENT SPECIFICATION

(11) **EP 3 826 585 B1**
(45) Date of publication and mention of the grant of the patent: **01.10.2025**
(21) Application number: 19749531.0
(22) Date of filing: 23.07.2019
(51) Int. Cl.: A61F 2/07, A61F 2/06

(54) **FLOW RESTRICTING STENT-GRAFT**
FLUSSBEGRENZENDES STENTTRANSPLANTAT
ENDOPROTHÈSE VASCULAIRE LIMITANT L'ÉCOULEMENT

(30) Priority: 24.07.2018 US 201862702700 P
(43) Date of publication of application: 02.06.2021
(73) Proprietor: W. L. Gore & Associates, Inc., Newark, DE 19711 (US)
(72) Inventor: GOODMAN, Paul D., Newark, Delaware 19711 (US); JOHNSON, Dorthyann I., Newark, Delaware 19711 (US); TROKANSKI, Mark E., Newark, Delaware 19711 (US)
(74) Representative: HGF
(86) International application number: PCT/US2019/043039
(87) International publication number: WO 2020/023512

(56) References cited:
- EP-A1- 2 772 233
- WO-A1-2017/115267
- WO-A2-2017/154025
- CN-A- 107 440 817

## Description

### CROSS-REFERENCE TO RELATED APPLICATION

This application claims the benefit of Provisional Application No. 62/702,700, filed July 24, 2018.

### TECHNICAL FIELD

The present disclosure relates to systems, medical devices, and methods for treating heart failure and/or other cardiovascular diseases.

### BACKGROUND

Patients experiencing heart failure may have a buildup of excess fluid in the body. The excess fluid buildup may increase fluid accumulation in the interstitial space and worsen a patient's symptoms and quality of life. Excess fluid (or hypervolemia) is the leading cause of hospitalization for heart failure patients (approximately 1,000,000 per year in the United States).

Treatment of the excess fluid buildup may be treated pharmaceutically by diuretics (or other pharmaceutical agents). However, a patient may experience drug resistance, unwanted side effects, inappropriate dosing, or other issues such as failure to comply with medicine directives. Non-pharmaceutical options, such as implantable device solutions that provide an alternative to or augment pharmaceutical efficacy by influencing renal function, may be beneficial to avoid these and other issues in treatment of buildup of excess fluid in the body. Similarly, chronic high blood pressure (hypertension) can also be managed pharmaceutically by diuretics (or other antihypertensive pharmaceutical agents). In addition, other disease states may result in hypotension, reduced cardiac output, and poor renal function. Insofar as the kidneys play a central role in regulating systemic blood pressure and fluid homeostasis, non-pharmaceutical options, such as implantable device solutions that provide an alternative to or augment pharmaceutical efficacy by influencing renal function, may provide an alternative means of managing the fluid imbalance resulting from chronic disease states such as heart failure, hypertension and other disease states.

Example implantable devices are described in European patent application EP 2772233 to The First Affiliated Hospital of Nanjing Medical University, International patent application WO 2017/115267 to Invatin Technologies Ltd., Chinese patent application CN107440817 to Beijing Saibo Medicine Tech Co Ltd, and International patent application WO 2017/154025 to Deshmukh et al.

### SUMMARY

The presently claimed invention is as defined in the appended set of claims. In one example ("Example 1"), an implantable medical device for altering blood flow in a vessel of a patient includes a first anchor portion and a second anchor portion configured to contact a vessel wall of the vessel; a flow restriction portion having a section of reduced diameter relative to the one or more anchor portions; and a membrane component coupled to only one side of the sections tapering inwardly of the flow restriction portion and configured to increase flow resistance within the main vessel and increase the blood flow into one or more side branches off the vessel.

In another example ("Example 2"), further to the device of Example 1, the membrane component is configured to increase flow into the one or more side branches off the vessel by between about 5% and about 30% and the side branches are proximal to the flow restriction portion.

In another example ("Example 3"), further to the device of any one of Examples 1-2, wherein the flow restriction portion form an hourglass shape, and the flow restriction portion tapers inwardly to reduce a diameter of the first anchor portion and the second anchor portion.

In another example ("Example 4"), further to the device of Example 3, the membrane component is coupled to one of the first anchor portion and the second anchor portion and another of the first anchor portion and the second anchor portion is uncovered.

In another example ("Example 5"), further to the device of Example 4, the membrane component is coupled to the first anchor portion to at least partially cover the first anchor portion to restrict blood flow laterally through the first anchor portion.

In another example ("Example 6"), further to the device of Example 5, the second anchor portion is uncovered and configured to allow blood flow laterally through the second anchor portion.

In another example ("Example 7"), further to the device of Example 6, the second anchor portion is configured to lessen opportunity for occluding the one or more side branches off the vessel.

In another example ("Example 8"), further to the device of any one of Examples 5-6, wherein the membrane component is coupled to the first anchor portion at a location where the first anchor portion is configured to contact the vessel wall.

In another example ("Example 9"), further to the device of Example 8, the location of the membrane component on the first anchor portion is configured to lessen thrombosis formation.

In another example ("Example 10"), further to the device of any one of Examples 1-9, the vessel is an aorta of the patient and the side branches are one or more of renal, celiac, hepatic, and mesenteric arteries, and the implantable medical device is configured to implant within an aorta and increase flow into one or more of the renal, celiac, the hepatic, and the mesenteric arteries.

In another example ("Example 11"), further to the device of Example 10, the membrane component is configured to increase blood pressure at an ostium of the at least one renal artery pressure across a kidney of the patient relative to venous outflow pressure causing more blood to flow through the kidney.

In another example ("Example 12"), further to the device of Example 11, the membrane component being configured to increase blood pressure at the ostium of the at least one renal artery facilitates increase of fluid filtration by the kidney to increase diuresis and lessen fluid retention of the patient.

In another example ("Example 13"), further to the device of any one of Examples 1-12, the membrane component is configured to induce stenosis of the vessel distal of the one or more side branches between about 40% and about 80%.

In another example ("Example 14"), further to the device of Example 13, the membrane component is configured to induce stenosis of the vessel distal of the one or more side branches between 50% and 70%.

In another example ("Example 15"), further to the device of any one of Examples 1-14, the first anchor portion or the second anchor portion is configured to oppose against the vessel wall in the vena cava and the membrane component is configured to create a narrowed flow lumen of the vena cava distal of one or both renal veins of between about 40% and about 90% to promote blood flow out of the kidney
In one example ("Example 16"), an implantable medical device for altering blood flow in a vessel of a patient includes a flow restriction section substantially blocking the blood flow laterally through the implantable medical device and reducing in diameter to restrict blood flow longitudinally within the vessel; and an open section configured to allow blood flow laterally through the implantable medical device.

In another example ("Example 17"), further to the device of Example 16, the flow restriction section includes a membrane component configured to increase flow resistance within the vessel and increase the blood flow into one or more side branches off the vessel.

In another example ("Example 18"), further to the device of Example 16, an open section is configured to facilitate blood flow into one or more side branches off the vessel without occluding the one or more side branches off the vessel.

In one example ("Example 19"), a method of altering blood flow in a vessel of a patient includes delivering an implantable medical device including a flow restriction section substantially blocking the blood flow laterally through the implantable medical device and reducing in diameter to restrict blood flow longitudinally within the vessel and an open section configured to allow blood flow laterally through the implantable medical device; and arranging the open section of the implantable medical device to at least partially overlap one or more side branches off the vessel to allow perfusion of blood flow into the one or more side branches.

In another example ("Example 20"), the method of Example 19, where arranging the open section of the implantable medical device includes arranging the closed section of the implantable medical device distal or proximal to the one or more side branches off the vessel.

The foregoing Examples are just that, and should not be read to limit or otherwise narrow the scope of any of the inventive concepts otherwise provided by the instant disclosure. While multiple examples are disclosed, still other embodiments will become apparent to those skilled in the art from the following detailed description, which shows and describes illustrative examples. Accordingly, the drawings and detailed description are to be regarded as illustrative in nature rather than restrictive in nature.

### BRIEF DESCRIPTION OF THE DRAWINGS

The accompanying drawings are included to provide a further understanding of the disclosure and are incorporated in and constitute a part of this specification, illustrate embodiments, and together with the description serve to explain the principles of the disclosure.
FIG. 1 shows an example implantable medical device in accordance with various aspects of the present disclosure.
FIG. 2 shows an example implantable medical device in accordance with various aspects of the present disclosure.
FIGS. 3A-B show an example implantable medical device implanted in a patient's vasculature accordance with various aspects of the present disclosure.
FIG. 4 show another example implantable medical device implanted in a patient's vessel in accordance with various aspects of the present disclosure.

### DETAILED DESCRIPTION

### Definitions and Terminology

This disclosure is not meant to be read in a restrictive manner. For example, the terminology used in the application should be read broadly in the context of the meaning those in the field would attribute such terminology.

With respect to terminology of inexactitude, the terms "about" and "approximately" may be used, interchangeably, to refer to a measurement that includes the stated measurement and that also includes any measurements that are reasonably close to the stated measurement. Measurements that are reasonably close to the stated measurement deviate from the stated measurement by a reasonably small amount as understood and readily ascertained by individuals having ordinary skill in the relevant arts. Such deviations may be attributable to measurement error, differences in measurement and/or manufacturing equipment calibration, human error in reading and/or setting measurements, minor adjustments made to optimize performance and/or structural parameters in view of differences in measurements associated with other components, particular implementation scenarios, imprecise adjustment and/or manipulation of objects by a person or machine, and/or the like, for example. In the event it is determined that individuals having ordinary skill in the relevant arts would not readily ascertain values for such reasonably small differences, the terms "about" and "approximately" can be understood to mean plus or minus 10% of the stated value.

### Description of Various Embodiments

Persons skilled in the art will readily appreciate that various aspects of the present disclosure can be realized by any number of methods and apparatuses configured to perform the intended functions. It should also be noted that the accompanying drawing figures referred to herein are not necessarily drawn to scale, but may be exaggerated to illustrate various aspects of the present disclosure, and in that regard, the drawing figures should not be construed as limiting.

Various aspects of the present disclosure are directed toward treating heart failure in a patient and/or other cardiovascular diseases such as hypertension and hypotension. In certain instances, the condition of the patient may deteriorate by buildup of excess fluid (*e.g*., hypervolemia) in the body. The buildup of fluid may increase fluid accumulation, principally in the tissues, and increase fluid and pressure in the various circulations and organs. The increased fluid and pressure in and of itself or in combination with an already failing heart may further harm the patient. As discussed in further detail below, various aspects of the present discourse are directed toward lessening buildup of excess fluid by use of an implantable medical device.

Various aspects of the disclosure are directed toward an implantable medical device configured to manipulate renal blood flow hemodynamics in order to induce a physiologically mediated therapeutic response. The implantable medical device discussed herein, in certain instances, is intended to increase natural diuresis and lessen buildup of excess fluid by increasing blood flow to the kidneys. By this action, this device is configured to redirect blood flow to the kidneys to reperfuse the kidneys, improve diuresis (increase fluid removal) and minimize/eliminate the impact of fluid overload on the heart. Kidney health may include the amount of injury that the kidney has sustained, is continuing to sustain, or a decrease in function relative to the baseline kidney function of a patient when healthy. In certain instances, kidney injury may be quantified by measuring Neutrophil gelatinase-associated lipocalin (NGAL).

The implantable medical devices, in certain instances, enable continuous and controlled fluid removal. As explained in further detail below, the patient's vasculature near the renal arteries is complex. More specifically, the patient's vasculature may include side branches from the aorta in addition to the renal arteries. The implantable medical devices may include a graft attached to at least a portion of a stent. The graft may be attached to only a portion of the stent to allow blood flow through the implantable medical device. The open or uncovered stent portions allow blood flow therethrough to enable positioning of the device without unintended blocking of side branch vessels.

In certain instances, the implantable medical devices discussed herein may be implanted in other vessels. The implantable medical devices may facilitate increase in peripheral resistance to treat decreases in blood pressure or resistance within the vasculature. As discussed further below, this may include implantation of the implantable medical devices for treatment of an arteriovenous (AV) fistula.

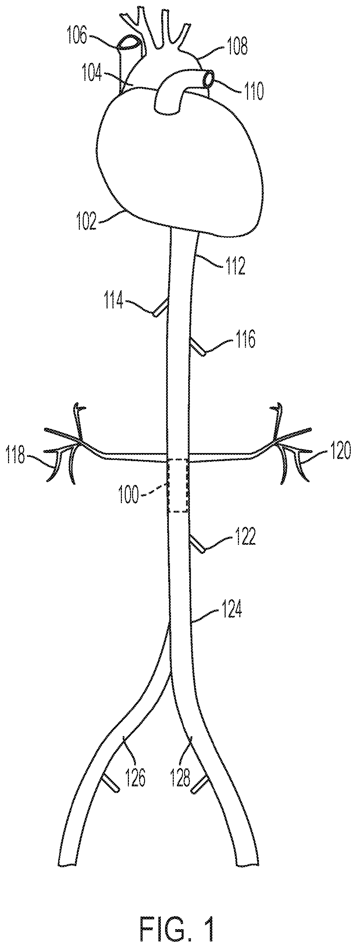

FIG. 1 shows an example implantable medical device 100 in accordance with various aspects of the present disclosure. The implantable medical device 100 is shown arranged within a patient's vasculature. The patient's vasculature shown in FIG. 1 includes the patient's heart 102, aortic root 104, superior vena cava 106, aortic arch 108, pulmonary trunk 110, descending aorta 112, celiac artery 114, superior mesenteric artery 116, renal arteries 118, 120, inferior mesenteric artery 122, abdominal aorta 124, and iliac arteries 126, 128. The implantable medical device 100 may be arranged within the aorta distal of the renal arteries 118, 120. In addition, the implantable medical device 100 may be configured to increase blood flow into at least one of the renal arteries 118, 120 while maintaining a substantially unrestricted blood flow within the aorta proximal to the renal arteries 118, 120. In addition, the implantable medical device 100 may be arranged within one or both of the iliac arteries 126, 128 in addition to or alternatively of the implantable medical device 100 being arranged within the aorta distal to the renal arteries 118, 120.

In certain instances, the implantable medical device 100 may be for augmenting perfusion of a branch vessel (*e.g.,* renal arteries 118, 120 or iliac arteries 126, 128) originating from the aorta. The implantable medical device 100 may be adjusted by increasing resistance to blood flow through the implantable medical device 100 to increase pressure within the aorta to increase blood flow into the branch vessel. In addition, the implantable medical device 100 may be configured to remain within the aorta for continuously augmenting perfusion.

The implantable medical device 100 being configured to increase blood flow into at least one of the renal arteries 118, 120 may reduce fluid accumulation by increasing the amount of blood that is filtered by the kidneys. In a patient suffering from heart failure, fluid overload may be caused (at least in part) by insufficient blood flow through the kidneys resulting from compromised cardiac output and venous congestion. Use of the implantable medical device 100 to increase blood flow into at least one of the renal arteries 118, 120 may increase kidney perfusion hemodynamically rather than pharmaceutically. To achieve increased kidney perfusion, resistance to blood flow distal to the renal arteries 118, 120 may be increased, which may decrease distal perfusion. The increased kidney perfusion enhances renal filtration and therefore removes fluid volume. In addition, the implantable medical device 100 may be used to enhance the performance of pharmacological treatments taken in connection therewith. For example, pharmacological treatments (*e.g.,* diuretics and/or hypertensive medications) may be enhanced by additionally enhancing the patient's kidney function.

In certain instances, the implantable medical device 100 being configured to increase blood flow into at least one of the renal arteries 118, 120 while maintaining a substantially unrestricted blood flow within the aorta proximal to the renal arteries 118, 120 may focus blood flow into the one or both of the renal arteries 118, 120. The restriction proximal to the renal arteries 118, 120 may direct blood flow to other areas supplied by the aorta such as the celiac artery 114, the superior mesenteric artery 116, or the brain. Thus, in certain instances, the implantable medical device 100 may be arranged within the aorta of the patient distal of (or overlapping) the renal arteries 118, 120. The result may be increased blood flow to at least one of the kidneys, by way of the increased blood flow to one or both of the renal arteries 118, 120, which may increase fluid removal from the circulation which would relieve the fluid and pressure accumulation in the various circulations and organs.

The implantable medical device 100 provides a non-pharmaceutical approach to increasing urine production (diuresis) and/or modifying systemic blood pressure. Patients may experience drug resistance, inaccurate dosing, or undesirable side effects. When drugs fail, aquapheresis or hemodialysis may be used to filter fluid directly from blood, however, these solutions are relatively invasive and disruptive to patient lifestyle and mobility. In addition, aquapheresis or hemodialysis may also produce hemodynamic instability with related cardiovascular complications, kidney damage, infection, and/or require capital equipment.

The implantable medical device 100 may change peripheral resistance when implanted percutaneously or surgically, temporarily or permanently, and may be adjustable to meet patient needs. The implantable medical device 100 may remain in the body after implantation for as long as the patient requires intervention. The implantable medical device 100 may be implanted for hours, days, or even years.

The illustrative implantable medical device 100 shown in FIG. 1 is not intended to suggest any limitation as to the scope of use or functionality of embodiments of the disclosure disclosed throughout this document. Neither should the illustrative implantable medical device 100 be interpreted as having any dependency or requirement related to any single component or combination of components illustrated therein. Additionally, any one or more of the components depicted in FIG. 1 can be, in embodiments, integrated with various ones of the other components depicted therein (and/or components not illustrated).

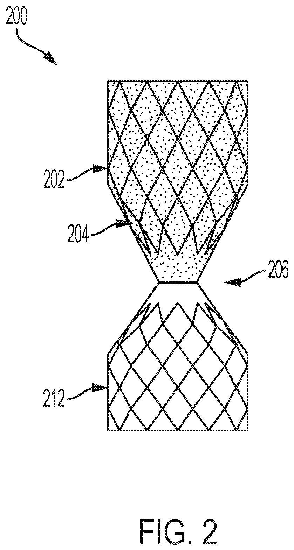

FIG. 2 shows an example implantable medical device 200 in accordance with various aspects of the present disclosure. The implantable medical device 200 is configured to alter blood flow in a vessel of a patient. The implantable medical device 200 includes one or more anchor portions include a first anchor portion 202 and a second anchor portion 212. The first anchor portion 202 and the second anchor portion 212 may include stent elements. In addition, the implantable medical device 200 may include a flow restriction portion 206 having a section of reduced diameter relative to the anchor portions 202, 212. As shown in FIG. 2, the flow restriction portion 206 tapers inwardly from one of the anchor portions 202, 212. In certain instances and as shown in FIG. 2, the anchor portions 202, 212 and the flow restriction portion 206 form an hourglass shape.

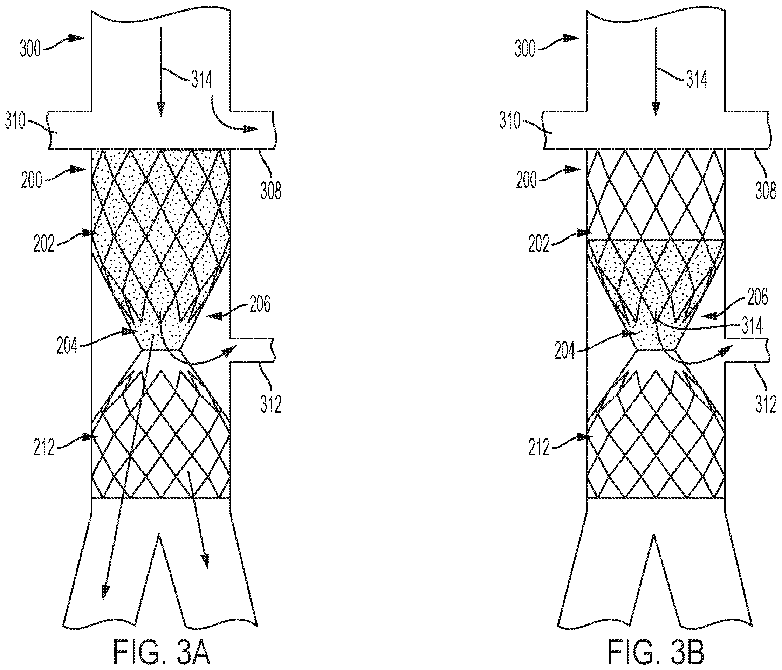

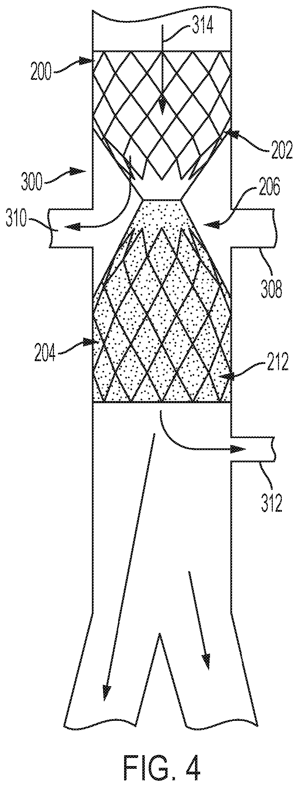

The implantable medical device may also include a membrane component 204 coupled to at least a portion of the flow restriction portion 206. The membrane component 204 is configured to increase flow resistance within a vessel and increase blood flow into one or more side branches off the vessel as explained in further detail with reference to FIGS. 3A-B and FIG. 4. The membrane component 204 is configured to increase flow into the one or more side branches off the vessel by between about 20% and about 30%. In addition, the membrane component 204 is coupled to one of the first anchor portion 202 and the second anchor portion 212 and another of the first anchor portion 202 and the second anchor portion 212 is uncovered. As shown in FIG. 2, the membrane component 204 is coupled to the first anchor portion 202. The membrane 206 at least partially covers the first anchor portion 202 to restrict blood flow laterally through the first anchor portion 202. In addition, the second anchor portion 212 is substantially uncovered to allow blood flow laterally through the second anchor portion 212.

The implantable medical device 200 may be configured to increase resistance and reduce blood flow distally thereof. The implantable medical device 200 may be diametrically adjustable. In certain instances, the implantable medical device 200 may be diameterically adjustable by way of a distensible force applied within the implantable medical device 200. The distensible force may be applied by way of a balloon structure. As noted above, the implantable medical device 200 may include a stent and a graft structure. Either or both of the stent and the graft structure of the implantable medical device 200 may be configured to maintain a new diameter in response to the distensible force. The stent portions of the implantable medical device 200 may be fabricated of Nitinol (NiTi) or stainless steel, and may be self-expanding or balloon expandable.

FIGS. 3A-B shows an example implantable medical device 200 implanted in a patient's vessel 300 in accordance with various aspects of the present disclosure. In certain instances, the implantable medical device 200 may be used in a method of altering blood flow 314 in a vessel of a patient. As shown in FIGS. 3A-B, the implantable medical device 200 is implanted within a vessel 300. The implantable medical device 200, which includes a stent element and a membrane component 204 attached to at least a portion of the stent element, may be delivered to a target location within the vessel 300 (*e.g.,* the aorta). The implantable medical device 200 is arranged to at least partially increase flow into one or more side branches 308, 310, 312 off the vessel (aorta) 300.

The implantable medical device 200 includes a first anchor portion 202 and a second anchor portion 212. The first anchor portion 202 and the second anchor portion 212 include stent elements. In addition, the implantable medical device 200 includes a flow restriction portion 206 having a section of reduced diameter relative to the anchor portions 202, 212. As shown in FIG. 3A-B, the flow restriction portion 206 tapers inwardly from one or both of the anchor portions 202, 212.

In certain instances and as shown in FIG. 2, the anchor portions 202, 212 and the flow restriction portion 206 form an hourglass shape. As shown in FIG. 2, the implantable medical device 200 tapers inwardly from both the first anchor portion 202 and the second anchor portion 212. The membrane component 204, however, is coupled to only one side of the sections tapering inwardly. The membrane component 204 is configured to increase flow resistance within a vessel and increase blood flow into one or more side branches off the vessel. Thus, the flow restriction portion 206 is considered to be the portion of the implantable medical device 200 having a reduced diameter relative to the anchor portions 202, 212 to which the membrane component 204 is attached or coupled to. Therefore, the flow restriction portion 206 of the implantable medical device 200 shown in FIGS. 3A-B is arranged adjacent to the first anchor portion 202. In other instances, the flow restriction portion 206 may be arranged adjacent to the second anchor portion 212 as shown in FIG. 4. In addition, the flow restriction portion 206 may be both portions of reduced diameter in instances where the membrane 204 is arranged on both portions of reduced diameter relative to the anchor portions 202, 212.

The membrane component 204 may be configured to increase flow into the one or more side branches 308, 310, 312 off the vessel by about 10% - about 20%, about 20% - about 30%, about 30% - about 40% or any number therebetween. In addition, the membrane component 204 is coupled to one of the first anchor portion 202 and the second anchor portion 212 and another of the first anchor portion 202 and the second anchor portion 212 is uncovered. As shown in FIG. 3A-B, the membrane component 204 is coupled to the first anchor portion 202. In addition, the membrane 206 may be coupled to an entirety of the first anchor portion 202 (as shown in FIG. 3A) or to a portion of the first anchor portion 202 (as shown in FIG. 3B). The second anchor portion 212 may be uncovered and configured to allow blood flow laterally through the second anchor portion 212.

The second anchor portion 212 is configured to facilitate blood flow into one or more side branches 308, 310, 312 off the vessel 300 without occluding the one or more side branches 308, 310, 312 off the vessel. As shown in FIGS. 3A and 3B, the flow restriction portion 206 of the implantable medical device 200 is a closed section substantially blocking the blood flow 314 laterally through the implantable medical device 200 and the second anchor portion 212 is an open section substantially allowing blood flow 314 laterally through the implantable medical device 200.

In certain instances, the implantable medical device 200 may produce a long-term or chronic physiological change in the patient. The implantable medical device 200 altering flow into the kidneys may produce a neuro-hormonal response that effects a change in the patient to move toward normal kidney functioning. The kidneys are a feedback regulator of systemic pressure through the patient's body. The implantable medical device 200 for altering flow into the kidneys provides a non-pharmaceutical means of influencing the kidneys' natural feedback mechanisms to regulate systemic pressure.

By adjusting the degree of hemodynamic alteration of renal perfusion, patient-specific adjustments to regulate blood pressure may be made. Adjusting the aortic flow resistance imparted by the implantable medical device 200, may influence renal artery pressure and/or flow rate, which, in turn, can manifest as transient or long-lasting alterations in systemic blood pressure. The changes induced by the implantable medical device 200, in renal-mediated blood pressure levels, may have therapeutic benefits in and of themselves. Likewise, changes induced by the implantable medical device 200 in renal-mediated blood pressure levels may be used in combination with various blood pressure medications to optimize blood pressure management on an individualized basis.

In certain instances, the implantable medical device 200 may increase a resistance to blood flow 314, within the aorta distal to the branches 308, 310 (*e.g.,* renal arteries), by between about 5% and about 30% as compared to normal flow. The implantable medical device 200 may occlude the aorta distal to the branches 308, 310 (*e.g.,* renal arteries) by between about 5% and about 30% to increase blood flow 314 into the kidneys. The positioning of the implantable medical device 200 may be altered to achieve between about 5% and about 30% increase of blood flow 314 into the kidneys. In addition, the amount of coverage and location of coverage of the membrane component 204 may be altered to achieve the desired blood flow.

In certain instances, the membrane component 204 is configured to induce stenosis of the aorta of the patient at least partially distal of the branches 308, 310 (renal arteries 118, 120) between about 40% and about 80% and alter blood flow 314 into one or more of the side branches 308, 310 of the vessel (aorta) 300 (*e.g.,* one or both of the renal arteries 118, 120) while maintaining a substantially unrestricted blood flow 314 within the aorta proximal to one or more of the side branches 308, 310 of the vessel (aorta) 300 and more particularly in the abdominal aorta (*e.g.,* one or both of the renal arteries 118, 120). In certain instances, the induced stenosis is between 50% and 70%. Clinically, measurement of ankle pressure, Doppler ultrasound velocity, ankle-brachial index, or other hemodynamic parameters in the lower limbs can be employed to optimize the magnitude of the induced stenosis while ensuring adequate limb perfusion. In addition, the membrane component 204 may be configured to increase blood pressure at an ostium of the at least one renal artery pressure across a kidney of the patient relative to venous outflow pressure causing more blood to flow through the kidney. The membrane component 204 may be configured to increase blood pressure at the ostium of the at least one renal artery facilitates increase of fluid filtration by the kidney to increase diuresis and lessen fluid retention of the patient.

When implanted in the aorta, the device 200 is configured to redirect blood flow into at least one of the renal arteries by diverting fluid within the aorta. To achieve increased kidney perfusion, resistance to blood flow distal to the renal arteries may be increased, which decreases distal perfusion. The increased kidney perfusion enhances renal production and therefore removes fluid volume. In certain instances, the device 200 is configured to create a narrowed flow lumen in the conduit of the aorta of the patient at least partially distal of the renal arteries between about 40% and about 80% and alter blood flow into at least one branch vessel of the aorta (e.g., one or both of the renal arteries). In certain instances, the induced restriction is between 50% and 70% of a nominal flow.

When implanted in the vena cava, the device 200 may augment perfusion from a tributary vessel (*e.g.,* renal veins) terminating in the vena cava by altering pressure within the vena cava to alter blood flow from the tributary vessel of the vena cava. In certain instances, the device 200 may be configured to create a narrowed flow lumen in the conduit located in the vena cava distal of the at least one tributary vessel of between about 40% and about 90%. Use of the flow restriction devices, discussed in further detail below, by dropping pressure in the renal veins may increase kidney perfusion hemodynamically rather than pharmaceutically.

In addition, the implantable medical device 200 may be implanted into another vessel of the patient that leads into an organ. In these instances, the membrane component 204 is configured to induce stenosis of the vessel into which the implantable medical device 200 is implanted distal of location at which the implantable medical device 200 is implanted between about 40% and about 80%. In addition, implanting the implantable medical device 200 in this manner alter blood flow 314 into the organ that the vessel leads into while maintaining a substantially unrestricted blood flow 314 within the vessel proximal to the location of implantation.

In certain instances, the implantable medical device 200 is arranged in a vessel other than the aorta or venal cava. In these instances, the flow altering element 208 may be configured to alter the blood flow through the lumen to restrict blood flow in the vessel and induce a physiologically mediated therapeutic response in the patient. In certain instances, the implantable medical device 200 is configured to induce the physiologically mediated therapeutic response to include an increase in peripheral resistance within the vessel. The implantable medical device 200 may be configured to treat a fistula within the vessel and increase in peripheral resistance within the vessel as described in further detail below.

Further, as shown in FIG. 3A-B, the membrane component 204 may be coupled to the first anchor portion 202 at a location where the first anchor portion 202 is configured to contact the vessel wall. In these instances, the location of the membrane component 204 on the first anchor portion 202 is configured to lessen thrombosis on the vessel wall. The membrane component 204 may additionally allow continuous (retrograde or back) flow through the flow restriction portion 206 to lessen the opportunity for blood to stagnate, which can result in clotting. More specifically, blood flows external to the flow restriction portion 206 to lessen the opportunity for clotting.

As shown in FIG. 3A-B, the flow restriction portion 206 is arranged adjacent to a side branch vessel 312 into which an increased blood flow is desired. In certain instances, the flow restriction portion 206 is arranged upstream from the side branch vessel 312 into which an increased blood flow is desired. The second anchor portion 212 (open section) may be arranged laterally adjacent to the side branch vessel 312 into which an increased blood flow is desired to allow blood flow laterally through the second anchor portion 212 without occluding the side branch vessel 312.

FIG. 4 show another example implantable medical device 200 implanted in a patient's vessel in accordance with various aspects of the present disclosure. The implantable medical device 200, which includes a stent element and a membrane component 204 attached to at least a portion of the stent element, may be delivered to a target location within the vessel 300 (*e.g.,* the aorta). The implantable medical device 200 is arranged to at least partially increase flow into one or more side branches 308, 310, 312 off the vessel (aorta) 300.

The implantable medical device 200 includes a first anchor portion 202 and a second anchor portion 212. The first anchor portion 202 and the second anchor portion 212 may include stent elements. In addition, the implantable medical device 200 may include a flow restriction portion 206 having a section of reduced diameter relative to the anchor portions 202, 212. As shown in FIG. 4, the flow restriction portion 206 tapers inwardly from one of the anchor portions 202, 212.

The membrane component 204, however, is coupled to only one side of the sections tapering inwardly. The membrane component 204 may be configured to increase flow resistance within a vessel and increase blood flow into one or more side branches off the vessel. Thus, the flow restriction portion 206 is considered to be the portion of the implantable medical device 200 having a reduced diameter relative to the anchor portions 202, 212 to which the membrane component 204 is attached or coupled to. Therefore, the flow restriction portion 206 of the implantable medical device 200 shown in FIG. 4 is arranged adjacent to the second anchor portion 212.

As shown in FIG. 4, the second anchor portion 212 is a closed section substantially blocking the blood flow 314 laterally through the implantable medical device 200 and the first anchor portion 202 is an open section substantially allowing blood flow 314 radially through the implantable medical device 200. As shown comparing the implantable medical devices 200 in FIGS. 3A-B and FIG. 4, the flow restriction portion 206 may partially cover the side branch vessel 308, 310 into which an increased blood flow is desired. In certain instances, the flow restriction portion 206 is arranged at least partially downstream from the side branch vessel 308, 310 into which an increased blood flow is desired. The first anchor portion 202 (open section) may be arranged laterally adjacent to the side branch vessel 308, 310 into which an increased blood flow is desired to allow blood flow laterally through the first anchor portion 202 without occluding the side branch vessel 308, 310.

In certain instances, patients with heart failure (such as late-stage heart failure) may have an elevated sympathetic nervous system state in part due to decreased cardiac output (blood pressure and flow in one of both of the kidneys). One compensatory output of this state is to generate a signal to attempt to preserve cardiac output, which puts further strain (myocardial oxygen demand) on the heart. Implantable medical devices discussed herein, and the methods that include the implantable medical devices, are directed toward increasing the pressure (mean or peak systolic) in the kidney to reduce stimulation of the neuro-hormonal response (*e.g.,* decrease in the sympathetic activation nervous system). The result of the reduced activation of the sympathetic nervous system, by way of the implantable medical device or methods that include the implantable medical device, may decrease resting heart rate and blood pressure.

Further and in certain instances, patients with heart failure (such as late-stage heart failure) may have activation of the Renin-Angiotensin-Aldosterone system (RAAS), in part due to decreased cardiac output resulting in impaired blood flow to the kidney. A consequence of the elevated RAAS is to generate a signal that stimulates adverse myocardial structural changes. Implantable medical devices discussed herein, and the methods that include the implantable medical devices, are directed toward increasing the pressure (mean or peak systolic) in the kidney to reduce stimulation of the RAAS. The result of the reduced activation of the RAAS, by way of the implantable medical device or methods that include the implantable medical device, may be a reduced sympathetic nervous system activation and attenuation of adverse cardiac remodeling.

Previous studies of implantable medical devices implanted in the aorta have been used to evaluate response of canines with induced heart failure (coronary microembolization resulting in ejection fraction of about 30%). Hemodynamic status observed in the test group relative to the control group indicated improved cardiac function and decreased sympathetic nervous system tone. For example, heart rate and mean arterial pressure decreased, while contractility increased relative to controls. These comparative outcomes were supported by positive shifts in biomarkers such as pro-BNP and NGAL, relative to controls. As an example, animals with implant produced about 35% more urine with about 21% higher creatinine content, resulting in about 52% less increase in serum creatinine as a result of the diuretic challenge. These results show that an implantable medical device, such an implantable medical device directing blood into the kidneys or restricting blood flow within the aorta distal to the renal arteries, placed in the aorta may help decrease the symptoms of fluid overload and cardiac stress associated with heart failure. The devices are shown to increase blood pressure proximal to the stenosis and, in doing so, increase kidney perfusion pressure, thus increasing kidney perfusion. A secondary effect of this device is the reduction in the activation of the RAAS system. Effectiveness of the device was based on assessment of central hemodynamics, left ventricular (LV) function and renal function.

In addition, previous studies have found that induced stenosis has little effect on flow or pressure until it reaches about 40%, after which the impact is dependent on artery diameter and blood flow rate. Based on the above animal study, however, it has been discovered that there is a threshold above which the impact dramatically increases. The regime for stenosis, based on these results, is between about 40% and about 80%, and more particularly between about 50% and about 70%. Clinically, measurement of ankle pressure, Doppler ultrasound velocity, or other hemodynamic parameters in the lower limbs can be employed to optimize the magnitude of the induced stenosis while ensuring adequate limb perfusion.

In addition and in certain instances, the devices discussed above describe diametric restriction of the aorta or vena cava, however, a length of the restriction portion also may affect the amount of restriction in the aorta or vena cava. Thus, the length of the restriction portion and the diameter or circumference of the restriction portion may be varied to achieve a desired stenosis or restriction percentage.

Further, the devices discussed herein may implanted within vessels for treatment of an arteriovenous (AV) fistula. AV fistula formation may lead to a decrease in peripheral resistance within the vasculature. Implantation of the devices discussed herein at or adjacent to the AV fistula may increase flow resistance to a nominal level and counteract the decrease in peripheral resistance resulting from the AV fistula. In certain instances, the devices are implanted distal to the AV fistula, proximal to the AV fistula, or across the AV fistula.

Examples of synthetic polymers (which may be used as a graft component coupled to at least a portion of the stent element(s)) include, but are not limited to, nylon, polyacrylamide, polycarbonate, polyformaldehyde, polymethylmethacrylate, polytetrafluoroethylene, polytrifluorochlorethylene, polyvinylchloride, polyurethane, elastomeric organosilicon polymers, polyethylene, polypropylene, polyurethane, polyglycolic acid, polyesters, polyamides, their mixtures, blends and copolymers are suitable as a membrane material. In one embodiment, the membrane material is made from a class of polyesters such as polyethylene terephthalate including DACRON^{®} and MYLAR ^{®} and polyaramids such as KEVLAR^{®}, polyfluorocarbons such as polytetrafluoroethylene (PTFE) with and without copolymerized hexafluoropropylene (TEFLON^{®} or GORE-TEX^{®}), and porous or nonporous polyurethanes. In certain instances, the membrane material comprises expanded fluorocarbon polymers (especially PTFE) materials described in British. Pat. No. 1,355,373; 1,506,432; or 1,506,432 or in U.S. Pat. No. 3,953,566; 4,187,390; or 5,276,276. Included in the class of preferred fluoropolymers are polytetrafluoroethylene (PTFE), fluorinated ethylene propylene (FEP), copolymers of tetrafluoroethylene (TFE) and perfluoro(propyl vinyl ether) (PFA), homopolymers of polychlorotrifluoroethylene (PCTFE), and its copolymers with TFE, ethylene-chlorotrifluoroethylene (ECTFE), copolymers of ethylene-tetrafluoroethylene (ETFE), polyvinylidene fluoride (PVDF), and polyvinyfluoride (PVF). Especially preferred, because of its widespread use in vascular prostheses, is ePTFE. In certain instances, the membrane material comprises a combination of said materials listed above.

Additional examples of graft materials include, but are not limited to, vinylidinefluoride/hexafluoropropylene hexafluoropropylene (HFP), tetrafluoroethylene (TFE), vinylidenefluoride, 1-hydropentafluoropropylene, perfluoro(methyl vinyl ether), chlorotrifluoroethylene (CTFE), pentafluoropropene, trifluoroethylene, hexafluoroacetone, hexafluoroisobutylene, fluorinated poly(ethylene-co-propylene (FPEP), poly(hexafluoropropene) (PHFP), poly(chlorotrifluoroethylene) (PCTFE), poly(vinylidene fluoride (PVDF), poly(vinylidene fluoride-co-tetrafluoroethylene) (PVDF-TFE), poly(vinylidene fluoride-co-hexafluoropropene) (PVDF-HFP), poly(tetrafluoroethylene-co-hexafluoropropene) (PTFE-HFP), poly(tetrafluoroethylene-co-vinyl alcohol) (PTFE-VAL), poly(tetrafluoroethylene-co-vinyl acetate) (PTFE-VAC), poly(tetrafluoroethylene-co-propene) (PTFEP) poly(hexafluoropropene-co-vinyl alcohol) (PHFP-VAL), poly(ethylene-co-tetrafluoroethylene) (PETFE), poly(ethylene-co-hexafluoropropene) (PEHFP), poly(vinylidene fluoride-co-chlorotrifluoroe-thylene) (PVDF-CTFE), and combinations thereof, and additional polymers and copolymers described in U.S. Publication 2004/0063805. Additional polyfluorocopolymers include tetrafluoroethylene (TFE)/perfluoroalkylvinylether (PAVE). PAVE can be perfluoromethylvinylether (PMVE), perfluoroethylvinylether (PEVE), or perfluoropropylvinylether (PPVE), as essentially described in U.S. Publication 2006/0198866 and U.S. Pat. No. 7,049,380. Other polymers and copolymers include, polylactide, polycaprolacton-glycolide, polyorthoesters, polyanhydrides; poly-aminoacids; polysaccharides; polyphosphazenes; poly(ether-ester) copolymers, e.g., PEO-PLLA, or blends thereof, polydimethyl-siolxane; poly(ethylene-vingylacetate); acrylate based polymers or copolymers, e.g., poly(hydroxyethyl methylmethacrylate, polyvinyl pyrrolidinone; fluorinated polymers such as polytetrafluoroethylene; cellulose esters and any polymer and copolymers described in U.S. Publication 2004/0063805.

The graft components, as discussed herein, may be attached to the self-expanding stent elements by using a coupling member that is generally a flat ribbon or tape having at least one generally flat surface. In certain instances, the tape member is made from expanded PTFE (ePTFE) coated with an adhesive. The adhesive may be a thermoplastic adhesive. In certain instances, the thermoplastic adhesive may be fluorinated ethylene propylene (FEP). More specifically, an FEP-coated side of the ePTFE may face toward and contacts an exterior surface of the self-expanding stent and graft component, thus attaching the self-expanding stent to the graft component. Materials and method of attaching a stent to the graft is discussed in U.S. Pat. No. 6,042,602 to Martin.

The stent elements discussed herein can be fabricated from a variety of biocompatible materials. These materials may include 316L stainless steel, cobalt-chromium-nickel-molybdenum-iron alloy ("cobalt-chromium"), other cobalt alloys such as L605, tantalum, nickel-titanium alloys (e.g., Nitinol), or other biocompatible metals. In certain instances, as discussed in detail above, the stent (and graft) may be self-expanding. The prosthesis may be balloon expandable

A variety of materials variously metallic, super elastic alloys, such as Nitinol, are suitable for use in these stents. Primary requirements of the materials are that they be suitably springy even when fashioned into very thin sheets or small diameter wires. Various stainless steels which have been physically, chemically, and otherwise treated to produce high springiness are suitable as are other metal alloys such as cobalt chrome alloys (*e.g.,* ELGILOY^{®}), platinum/tungsten alloys, and especially the nickel-titanium alloys (*e.g.,* Nitinol).

## Claims

1. An implantable medical device (200) for altering blood flow in a vessel of a patient, the implantable medical device (200) comprising:
a first anchor portion (202) and a second anchor portion (212) configured to contact a vessel wall of the vessel;
a flow restriction portion (206) having a section of reduced diameter relative to the first and second anchor portions (202, 212) such that the flow restriction portion (206) tapers inwardly from the first and second anchor portions (202, 212); **characterized in that**
a membrane component (204) is coupled to only one side of the sections tapering inwardly of the flow restriction portion (206) and configured to increase flow resistance within the main vessel and increase the blood flow into one or more side branches off the vessel.

2. The device (200) of claim 1, wherein the membrane component (204) is configured to increase flow into the one or more side branches off the vessel by between about 5% and about 30% and the side branches are proximal to the flow restriction portion (206).

3. The device (200) of any one of claims 1-2, wherein the flow restriction portion (206) forms an hourglass shape.

4. The device (200) of claim 3, wherein the membrane component (204) is coupled to one of the first anchor portion (202) and the second anchor portion (212) and another of the first anchor portion (202) and the second anchor portion (212) is uncovered.

5. The device (200) of claim 4, wherein the membrane component (204) is coupled to the first anchor portion (202) to at least partially cover the first anchor portion (202) to restrict blood flow laterally through the first anchor portion (202).

6. The device (200) of claim 5, wherein the second anchor portion (212) is uncovered and configured to allow blood flow laterally through the second anchor portion (212).

7. The device (200) of claim 6, wherein the second anchor portion (212) is configured to lessen opportunity for occluding the one or more side branches off the vessel.

8. The device (200) of any one of claims 5-6, wherein the membrane component (204) is coupled to the first anchor portion (202) at a location where the first anchor portion (202) is configured to contact the vessel wall.

9. The device (200) of claim 8, wherein the location of the membrane component (204) on the first anchor portion (202) is configured to lessen thrombosis formation.

10. The device (200) of any one of claims 1-9, wherein vessel is an aorta of the patient and the side branches are one or more of renal, celiac, hepatic, and mesenteric arteries, and the implantable medical device (200) is configured to implant within an aorta and increase flow into one or more of the renal, celiac, the hepatic, and the mesenteric arteries, wherein the membrane component (204) is configured to increase blood pressure at an ostium of the at least one renal artery pressure across a kidney of the patient relative to venous outflow pressure causing more blood to flow through the kidney.

11. The device (200) of claim 10, wherein the membrane component (204) being configured to increase blood pressure at the ostium of the at least one renal artery facilitates increase of fluid filtration by the kidney to increase diuresis and lessen fluid retention of the patient.

12. The device (200) of any one of claims 1-11, wherein the membrane component (204) is configured to induce stenosis of the vessel distal of the one or more side branches between about 40% and about 80%.

13. The device (200) of claim 12, wherein the membrane component (204) is configured to induce stenosis of the vessel distal of the one or more side branches between 50% and 70%.

14. The device (200) of any one of claims 1-13, wherein the first anchor portion (202) or the second anchor portion (212) is configured to oppose against the vessel wall in the vena cava and the membrane component (204) is configured to create a narrowed flow lumen of the vena cava distal of one or both renal veins of between about 40% and about 90% to promote blood flow out of the kidney.

## Patentansprüche

1. Implantierbare medizinische Vorrichtung (200) zum Ändern von Blutfluss in einem Gefäß eines Patienten, wobei die implantierbare medizinische Vorrichtung (200) Folgendes umfasst:
einen ersten Ankerabschnitt (202) und einen zweiten Ankerabschnitt (212), die konfiguriert sind, um eine Gefäßwand des Gefäßes zu kontaktieren;
einen Flussbegrenzungsabschnitt (206) mit einem Teil mit reduziertem Durchmesser relativ zu dem ersten und dem zweiten Ankerabschnitt (202, 212), sodass sich der Flussbegrenzungsabschnitt (206) von dem ersten und dem zweiten Ankerabschnitt (202, 212) nach innen verjüngt;
**dadurch gekennzeichnet, dass** eine Membrankomponente (204) an nur eine Seite der Teile gekoppelt ist, die sich von dem Flussbegrenzungsabschnitt (206) nach innen verjüngen und konfiguriert sind, um Flusswiderstand innerhalb des Hauptgefäßes zu erhöhen und den Blutfluss in einen oder mehrere Seitenzweige von dem Gefäß zu erhöhen.

2. Vorrichtung (200) nach Anspruch 1, wobei die Membrankomponente (204) konfiguriert ist, um Fluss in den einen oder die mehreren Seitenzweige von dem Gefäß um zwischen etwa 5 % und etwa 30 % zu erhöhen und die Seitenzweige proximal zu dem Flussbegrenzungsabschnitt (206) sind.

3. Vorrichtung (200) nach einem der Ansprüche 1-2, wobei der Flussbegrenzungsabschnitt (206) eine Sanduhrform bildet.

4. Vorrichtung (200) nach Anspruch 3, wobei die Membrankomponente (204) an einen von dem ersten Ankerabschnitt (202) und dem zweiten Ankerabschnitt (212) gekoppelt ist und ein anderer von dem ersten Ankerabschnitt (202) und dem zweiten Ankerabschnitt (212) unbedeckt ist.

5. Vorrichtung (200) nach Anspruch 4, wobei die Membrankomponente (204) an den ersten Ankerabschnitt (202) gekoppelt ist, um den ersten Ankerabschnitt (202) zumindest teilweise zu bedecken, um Blutfluss lateral durch den ersten Ankerabschnitt (202) zu beschränken.

6. Vorrichtung (200) nach Anspruch 5, wobei der zweite Ankerabschnitt (212) unbedeckt und konfiguriert ist, um Blutfluss lateral durch den zweiten Ankerabschnitt (212) zu ermöglichen.

7. Vorrichtung (200) nach Anspruch 6, wobei der zweite Ankerabschnitt (212) konfiguriert ist, um Gelegenheit zum Verschließen des einen oder der mehreren Seitenzweige von dem Gefäß zu verringern.

8. Vorrichtung (200) nach einem der Ansprüche 5-6, wobei die Membrankomponente (204) an den ersten Ankerabschnitt (202) an einer Stelle gekoppelt ist, an welcher der erste Ankerabschnitt (202) konfiguriert ist, um die Gefäßwand zu kontaktieren.

9. Vorrichtung (200) nach Anspruch 8, wobei die Stelle der Membrankomponente (204) an dem ersten Ankerabschnitt (202) konfiguriert ist, um Thrombosebildung zu verringern.

10. Vorrichtung (200) nach einem der Ansprüche 1-9, wobei Gefäß eine Aorta des Patienten ist und die Seitenzweige eines oder mehrere von Nieren-, Zöliakie-, Leber- und Mesenterialarterien sind, und die implantierbare medizinische Vorrichtung (200) konfiguriert ist, um innerhalb einer Aorta zu implantieren und Fluss in eine oder mehrere von den Nieren-, Zöliakie-, den Leber- und den Mesenterialarterien zu erhöhen, wobei die Membrankomponente (204) konfiguriert ist, um Blutdruck an einem Ostium des zumindest einen Nierenarteriendrucks über eine Niere des Patienten relativ zu venösem Ausflussdruck zu erhöhen, wodurch bewirkt wird, dass mehr Blut durch die Niere fließt.

11. Vorrichtung (200) nach Anspruch 10, wobei die Membrankomponente (204), die konfiguriert ist, um Blutdruck an dem Ostium der zumindest einen Nierenarterie zu erhöhen, Erhöhung von Fluidfiltration durch die Niere erleichtert, um Diurese zu erhöhen und Fluidretention des Patienten zu verringern.

12. Vorrichtung (200) nach einem der Ansprüche 1-11, wobei die Membrankomponente (204) konfiguriert ist, um Stenose des Gefäßes distal von dem einen oder den mehreren Seitenzweigen zwischen etwa 40 % und etwa 80 % zu induzieren.

13. Vorrichtung (200) nach Anspruch 12, wobei die Membrankomponente (204) konfiguriert ist, um Stenose des Gefäßes distal von dem einen oder den mehreren Seitenzweigen zwischen 50 % und 70 % zu induzieren.

14. Vorrichtung (200) nach einem der Ansprüche 1-13, wobei der erste Ankerabschnitt (202) oder der zweite Ankerabschnitt (212) konfiguriert ist, um der Gefäßwand in der Hohlvene gegenüberzuliegen und die Membrankomponente (204) konfiguriert ist, um ein verengtes Flusslumen der Hohlvene distal von einer oder beiden Nierenvenen zwischen etwa 40 % und etwa 90 % zu erzeugen, um Blutfluss aus der Niere zu fördern.

## Revendications

1. Dispositif médical implantable (200) permettant de modifier l'écoulement de sang dans un vaisseau d'un patient, le dispositif médical implantable (200) comprenant :
une première partie d'ancrage (202) et une seconde partie d'ancrage (212) conçues pour venir en contact avec une paroi de vaisseau du vaisseau ;
une partie de limitation d'écoulement (206) comportant une section de diamètre réduit par rapport aux première et seconde parties d'ancrage (202, 212) de sorte que la partie de limitation d'écoulement (206) se rétrécisse vers l'intérieur à partir des première et seconde parties d'ancrage (202, 212) ;
**caractérisé en ce qu'**un composant de membrane (204) est couplé à un seul côté des sections se rétrécissant vers l'intérieur de la partie de limitation d'écoulement (206) et conçue pour augmenter la résistance à l'écoulement à l'intérieur du vaisseau principal et augmenter le l'écoulement de sang dans une ou plusieurs ramifications latérales hors du vaisseau.

2. Dispositif (200) selon la revendication 1, ledit composant de membrane (204) étant conçu pour augmenter l'écoulement dans lesdites une ou plusieurs ramifications latérales hors du vaisseau d'environ 5 % à environ 30 % et lesdites ramifications latérales étant proximales par rapport à la partie de limitation d'écoulement (206).

3. Dispositif (200) selon l'une quelconque des revendications 1 à 2, ladite partie de limitation d'écoulement (206) présentant la forme d'un sablier.

4. Dispositif (200) selon la revendication 3, ledit composant de membrane (204) étant couplé à une partie parmi la première partie d'ancrage (202) et la seconde partie d'ancrage (212) et l'autre partie parmi la première partie d'ancrage (202) et la seconde partie d'ancrage (212) étant non recouverte.

5. Dispositif (200) selon la revendication 4, ledit composant de membrane (204) étant couplé à la première partie d'ancrage (202) de manière à recouvrir au moins partiellement la première partie d'ancrage (202) afin de limiter l'écoulement de sang latéral à travers la première partie d'ancrage (202).

6. Dispositif (200) selon la revendication 5, ladite seconde partie d'ancrage (212) étant non recouverte et conçue pour permettre un écoulement de sang latéral à travers la seconde partie d'ancrage (212).

7. Dispositif (200) selon la revendication 6, ladite seconde partie d'ancrage (212) étant conçue pour réduire les possibilités d'occlusion desdites une ou plusieurs ramifications latérales hors du vaisseau.

8. Dispositif (200) selon l'une quelconque des revendications 5 à 6, ledit composant de membrane (204) étant couplé à la première partie d'ancrage (202) au niveau d'un emplacement dans lequel la première partie d'ancrage (202) est conçue pour venir en contact avec la paroi du vaisseau.

9. Dispositif (200) selon la revendication 8, ledit emplacement du composant de membrane (204) sur la première partie d'ancrage (202) étant conçu pour réduire la formation de thrombose.

10. Dispositif (200) selon l'une quelconque des revendications 1 à 9, ledit vaisseau étant une aorte du patient et lesdites ramifications latérales étant une ou plusieurs artères parmi les artères rénales, cœliaques, hépatiques et mésentériques, ledit dispositif médical implantable (200) étant conçu pour s'implanter à l'intérieur d'une aorte et augmenter l'écoulement dans une ou plusieurs artères parmi les artères rénales, cœliaques, hépatiques et mésentériques, ledit composant de membrane (204) étant conçu pour augmenter la pression sanguine au niveau d'un ostium de ladite au moins une pression d'artère rénale à travers un rein du patient par rapport à la pression d'écoulement veineux provoquant l'écoulement de plus de sang à travers le rein.

11. Dispositif (200) selon la revendication 10, ledit composant de membrane (204) conçu pour augmenter la pression sanguine au niveau de l'ostium de ladite au moins une artère rénale facilitant l'augmentation de la filtration de fluide par le rein de manière à augmenter la diurèse et à réduire la rétention de fluide du patient.

12. Dispositif (200) selon l'une quelconque des revendications 1 à 11, ledit composant de membrane (204) étant conçu pour induire une sténose du vaisseau distal par rapport auxdites une ou plusieurs ramifications latérales entre environ 40 % et environ 80 %.

13. Dispositif (200) selon la revendication 12, ledit composant de membrane (204) étant conçu pour induire une sténose du vaisseau distal par rapport auxdites une ou plusieurs ramifications latérales entre 50 % et 70 %.

14. Dispositif (200) selon l'une quelconque des revendications 1 à 13, ladite première partie d'ancrage (202) ou ladite seconde partie d'ancrage (212) étant conçue pour s'opposer à la paroi du vaisseau dans la veine cave et ledit composant de membrane (204) étant conçu pour créer une lumière d'écoulement rétrécie de la veine cave distale par rapport à l'une et/ou l'autre veine rénale entre environ 40 % et environ 90 % de manière à favoriser l'écoulement de sang hors du rein.
